# EUROPEAN PATENT SPECIFICATION

(11) **EP 2 375 249 B1**
(45) Date of publication and mention of the grant of the patent: **25.12.2019**
(21) Application number: 11160450.0
(22) Date of filing: 30.03.2011
(51) Int. Cl.: G01N 33/49, B01L 3/00

(54) **Devices and process for separating plasma from a blood sample**
Vorrichtungen und Verfahren zum Trennen von Plasma aus einer Blutprobe
Dispositifs et procédé de séparation de plasma d'un échantillon sanguin

(30) Priority: 09.04.2010 EP 10159497
(43) Date of publication of application: 12.10.2011
(73) Proprietor: F. Hoffmann-La Roche AG, 4070 Basel (CH); Roche Diagnostics GmbH, 68305 Mannheim (DE)
(72) Inventor: Heinrich, Michael, 8001, Zürich (CH); Sandoz, Roger, 6364, Rotkreuz (CH); Wahl, Hans-Peter, 79650, Schopfheim (DE); Sarofim, Emad, 6332, Hagendorn (CH); Gruebl, Tomas, 6004, Luzern (CH)
(74) Representative: Peterreins Schley

(56) References cited:
- EP-A2- 0 436 897
- US-A- 4 839 296
- US-A- 6 106 732
- US-A1- 2005 209 532

## Description

### TECHNICAL FIELD

The present invention is in the field of clinical analysis and medical diagnostics and more particularly relates to devices and a process for separating plasma from a blood sample.

### BACKGROUND OF THE INVENTION

Blood analysis is commonly carried out on a sample of whole blood which for the majority of tests is drawn from the vein of the arm, the finger or the earlobe. A number of tests and procedures have been developed and many can be carried out simultaneously on one blood sample with such instruments as automatic analyzers. While most haematological tests relate to the blood cells, in daily routine, many tests are done on plasma or serum instead of the blood cells. Specifically, in recent years, an increasing number of immunochemical and nucleic acid analysis items can be observed. For instance, special tests can be used to detect substances contained in the plasma which are characteristic of specific infections such as HIV (Human Immunodeficiency Virus) particles. Accordingly, in view of performing such tests, there is an increasing need to separate plasma from the whole blood sample. Since these tests often involve sophisticated instruments, shipping of the plasma to specific analysis sites can be required.

The European patent EP 1096254 B1 describes a device for separating hematocrit from a whole blood sample provided with an inlet port for receiving the sample, a reaction region and a capillary pathway connecting the inlet port with the reaction region. The capillary pathway which is provided with obstructions for keeping the blood cells back is integrally formed with the reaction region.

Published European patent application EP 0 436 897 A2 discloses a device and method of separating the cellular components of whole blood from plasma or serum and assaying the plasma or serum for a soluble constituent. The device includes a filter pad that separates the cellular components of whole blood from the serum or plasma, in releasable contact with a test pad, that assays the serum or plasma for a particular soluble constituent. The filter pad, contaminated with the cellular components, is detachable from the plasma or serum-saturated test pad.

U.S. patent US 6,106,732 discloses devices including a sample-receiving element provided with a cover and a removable chipboard section positioned over a plasma sample retention pad.In light of the foregoing, it is an object of the invention to provide an improved device and process for separating plasma from a whole blood sample. These and further objects are met by devices and a process according to the independent claims. Preferred embodiments of the invention are given by the features of the dependent claims.

### SUMMARY OF THE INVENTION

The present invention relates to a device for separating plasma from a blood sample as set out in claim 1 and a process for separating plasma from a blood sample as set out in claim 13. Other embodiments are described in the dependent claims.

According to the invention, a new device for separating plasma from a whole blood sample including a partial volume of plasma and a partial volume of cellular components (hematocrit) is proposed. The device consists of a structure which usually, but not necessarily, is comprised of various functional layers, at least portions of which are stacked in an overlying relationship with respect to each other. The structure includes a first portion and a second portion in (or arranged to enable) fluid communication with the first portion and removably fixed thereto by means of at least one first adhesive element. In some embodiments, the first portion is in contact with the second portion.

Specifically, the first portion includes a separating member provided with a first surface, in the following denoted as "separating member surface", for applying the blood sample or receiving the blood sample from a take-up member as detailed below. The separating member is adapted to permit the passage of plasma and plasma macromolecules but to inhibit the passage of blood cells so as to separate the plasma from the cells when drawing blood through the separating member. Otherwise, the separating member is being adapted to provide free passage with respect to any specific analyte of interest the size of which is smaller than the typical size of cellular blood components such as but not limited to HIV (Human Immunodeficiency Virus) or any other kind of virus particles. In that, the separating member preferably includes a (chromatographic) depth filter element in series with a size-exclusion element, wherein the depth filter element slows the flow of blood cells relative to that of the plasma and the size-exclusion element permits plasma flow and blocks the passage of cellular blood components such as red and white blood cells and platelets. The depth filter element advantageously avoids clogging of the size-exclusion element and enables a lateral diffusion of the various components of blood so as to broaden the blood-contacted area of the second portion. The lateral diffusion can especially be useful in case of a lateral offset between the first and second portions.

The first portion preferably is a structural entity (unit) related to taking up the whole blood sample and separating the plasma from the blood sample.

Specifically, the second portion includes an absorptive member for absorbing plasma which is or can be brought in fluid communication with the separating member. More specifically, the absorptive member is provided with a second surface, in the following denoted as "absorptive member surface", which is or can be brought in contact with the separating member for receiving plasma from the separating member by means of capillary pressure generated by the absorptive member. Otherwise, the absorptive member is adapted for drying plasma contained therein so that the absorptive member may contain plasma in a wet or dried condition according to the specific demands of the user. Accordingly, the device of the invention allows for a capillary force-driven separation of plasma from the whole blood sample and absorption of the plasma by the absorptive member.

According to the present invention, the second portion includes a backing member such as a backing layer, preferably a solid (e.g. stiff) backing layer, e.g., arranged on one side of the absorptive member so as to structurally support, e.g. back, the absorptive member. Preferably, the absorptive member is sandwiched in-between the separating member and the backing member. The backing member can, for instance, include a recess for accommodating the absorptive member.

The second portion preferably is a structural entity (unit) related to absorbing the plasma and shipping the plasma to a dedicated analysis site preferably in a dried condition.

According to the invention, the first portion is (removably) fixed to the backing member by means of at least one first adhesive element in such a manner that the first portion can be removed from the backing member without destroying the absorptive member. Hence, the first portion can be removed from the backing member without destroying the absorptive member by selectively breaking the first adhesive element thus serving as predetermined breaking zone.

Specifically, in some embodiments, the first adhesive element is configured as an adhesive zone such as an adhesive spot or adhesive layer for fixing the first portion to the backing member. The first portion can, e.g., be fixed to the backing member in such a manner that it can be drawn away or peeled off from the backing member, e.g., backing layer.

In some embodiments, the first portion is fixed to a first supporting layer provided with an adhesive layer for fixing to the backing member, wherein the adhesive layer is adapted for peeling off the first supporting layer (together with the first portion) from the backing member. The first supporting layer can be provided on one or both sides with an adhesive layer consisting of adhesive material.

Thus, the first adhesive element advantageously allows for an easy and cost-effective fixation of the first portion to the backing member and removal therefrom without destroying the absorptive member.

According to the invention, the absorptive member is (removably) fixed to the backing member, e.g. backing layer, by means of at least one second adhesive element in such a manner that the absorptive member can be (non-destructively) removed from the backing member without destroying the absorptive member. Hence, the absorptive member can be removed from the backing member without destroying the absorptive member by selectively breaking the second adhesive element thus serving as predetermined breaking zone. Specifically, in some embodiments, the second adhesive element is configured as an adhesive zone such as an adhesive spot or adhesive layer for fixing the absorptive member to the backing member. In some embodiments, the absorptive member is fixed to the backing member in such a manner that it can be drawn away or peeled off from the backing member, e.g. backing layer.

In some embodiments, the absorptive member is fixed to a second supporting layer provided with an adhesive layer for fixing to the backing member, wherein the adhesive layer is adapted for peeling off the second supporting layer from the backing member. The second supporting layer can be provided on one or both sides with an adhesive layer consisting of adhesive material.

Thus, the second adhesive element advantageously allows for an easy and cost-effective fixation of the absorptive layer to the backing member and removal therefrom without destroying the absorptive member.

In some embodiments of the invention, the device includes at least one first gripping means such as a handle, adapted for manually or automatically removing the first portion from the backing member. Accordingly, the first portion can be readily removed from the backing member without a risk of contaminating the absorptive member by the user. Preferably, the first gripping means is fixed to the separating member. In some embodiments having a first supporting layer for fixing the first portion to the backing member, the first supporting layer is provided with a first gripping portion, preferably free of adhesive material, for gripping the first supporting layer so that the first portion can be readily removed from the backing member.

According to the present invention, the device includes at least one second gripping means such as a handle, adapted for removing the absorptive member from the backing member which facilitates handling of the absorptive member and advantageously avoids contamination of the absorptive member. The second gripping means is fixed to the absorptive member. In some embodiments having a second supporting layer for fixing the absorptive member to the backing member, the second supporting layer is provided with a second gripping portion, preferably free of adhesive material, for gripping the second supporting layer so that the absorptive member can be readily removed from the backing member.

As a result, in the device of the present invention, the absorptive member can be removed from the first portion without destroying the absorptive member. Hence, the absorptive member can be readily removed from the first portion to be dried and shipped in a cost-effective and easy manner at ambient temperatures without a need for plasma cooling. Since virus particles contained in the plasma normally lose their infectiousness in a dried state, a risk-less and safe transport of the absorptive member is possible. Specifically, the absorptive member can be safely shipped to an analysis site while being supported by the backing member and can be readily removed from the backing member at the analysis site facilitating plasma analysis.

In some embodiments, the first portion includes a take-up member for taking-up the whole blood sample which is in (or arranged to enable) fluid communication with the separating member so as to enable transport of the blood sample to the separating member. In some embodiments, the take-up member is in contact with the separating member. Contrary to the separating member, the take-up member is adapted to transport the blood without holding back cellular components contained therein. Specifically, the take-up member is provided with a third surface, in the following denoted as "take-up member surface", for applying the blood sample, wherein the separating member surface is in contact with the take-up member for receiving the blood sample from the take-up member by capillary pressure generated by the absorptive member. The take-up member advantageously allows for an easy capturing and storing of blood, preferably obtained from a patient's finger.

In some embodiments, the device further includes a cover member arranged on top of the first portion for covering at least a portion thereof provided with an opening for applying the blood sample to the take-up member surface or the separating member surface, respectively. The opening may be embodied as a mesh strip bonded to the cover member on both sides. In some embodiments, the cover member is fixed to the second portion. In some embodiments, the cover member is fixed to a bottom member arranged at the bottom-side of the second portion. In these embodiments, it is highly preferred that the take-up member is made of an elastically compressible material and arranged in-between the (non-elastic) cover member and the separating member in a (pre-)compressed condition so that the separating member is forced against the second portion and bottom member, respectively, by elastic decompression of the take-up member. The take-up member thus ensures a close fit with full contact for fluid communication between the various members of the device.

In some embodiments, the second portion in particular the backing member is being provided with a machine-readable label such as but not limited to a barcode which advantageously allows for an easy, quick and cost-effective identification of the absorptive member.

In some embodiments, the device of the invention further includes a humidity detector which being operatively coupled to the absorptive member can be used to detect humidity of the absorptive member. Specifically, the humidity detector preferably is adapted to output at least two optical and/or acoustic signals which are different with respect to each other in response to a detection result, wherein a first signal can be related to a dried condition (absence of liquid plasma) and a second signal can be related to a wet condition (presence of liquid plasma) of the absorptive member. The two signals preferably are different colour signals of an optical signal means.

In some embodiments, the separating member contains a plasma-soluble dye so that dye-coloured plasma can be optically identified in the absorptive member, preferably for checking plasma-saturation of the absorptive member.

In some embodiments, the absorptive member has a plasma capacity which is less than the amount of plasma in the blood volume contained in the separating member and/or take-up member so that the absorptive member becomes saturated with a predetermined amount of plasma when at least a portion of plasma contained therein is drawn into the absorptive member.

In some embodiments, the absorptive member is made of a material adapted to be dissolved in an elution medium for eluting dried plasma which advantageously allows for an easy and cost-effective elution of dried plasma contained in the absorptive member thus eliminating a risk of clotting in subsequent (e.g. automated) pipetting operations. Specifically, the material of the absorptive member may be chosen to specifically dissolve in the elution medium but not in the blood sample.

In some embodiments, the backing layer and/or the bottom layer is (are) adapted for keeping the absorptive element in a predetermined position with respect to the structure.

In some embodiments outside the scope of the present invention, the device is being made by conventional reel-to-reel technology wherein individual stripes or bands corresponding to the various members (layers) of the device are provided from feeding reels and then, following lamination of the bands, are wind-up by one target reel. Plural devices can readily be produced by cutting portions from the stacked band wind-up on the target reel thus enabling an easy and cost-effective way of producing the device in large numbers. Since those of skill in the art are aware of the reel-to-reel technology it is not further elucidated herein.

In some embodiments outside the scope of the present invention, one or more devices of the present invention are contained in an envelope preferably made of paper or papercard (cartone). The envelope preferably includes a bottom portion and two top portions connected to the bottom portion and arranged in opposite relationship with respect to each other. Specifically, the top portions are adapted to be opened or closed according to the specific demands of the user, wherein in closed positions the top portions overlap each other so that the envelope fully covers the one or more devices.

In some embodiments including a second adhesive element embodied as a supporting layer provided with an adhesive layer on one or both sides, wherein the supporting layer is being provided with a second gripping portion free of adhesive layer(s) for manually or automatically gripping the supporting layer, it is highly preferred that one of the top portions covers the second gripping portion so as to hide the second gripping portion in closed position of this top portion.

According to the invention, a new process according to claim 13 for separating plasma from a blood sample is proposed.

The above-described embodiments of the device and process of the invention may be used alone or in any combination thereof without departing from the scope of the invention.

### BRIEF DESCRIPTION OF THE DRAWINGS

Other and further objects, features and advantages of the invention will appear more fully from the following description. The accompanying drawings, which are incorporated in and constitute a part of the specification, illustrate preferred embodiments of the invention, and together with the general description given above and the detailed description given below, serve to explain the principles of the invention.
- FIG. 1: is a schematic sectional view illustrating an exemplary embodiment of the device according to the invention;
- FIG. 2: is a schematic sectional view illustrating another exemplary embodiment of the device according to the invention;
- FIG. 3: depicts a schematic sectional view and a smaller perspective view illustrating a yet another exemplary embodiment of the device according to the invention.

### DETAILED DESCRIPTION OF THE INVENTION

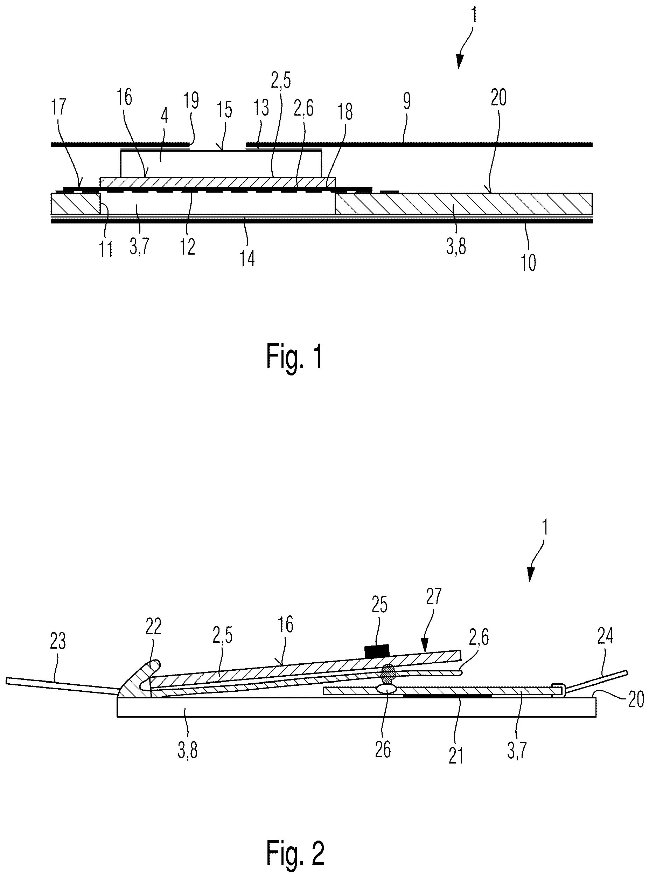

By way of illustration, specific exemplary embodiments in which the invention may be practiced are described. With reference to FIG. 1, by means of a schematic diagram, an exemplary embodiment of the device for separating plasma from a whole blood sample is explained.

Accordingly, a device for separating plasma from a whole blood sample generally referred to at reference numeral 1 includes a stacked structure comprised of various functional layers stacked in a parallel, overlying relationship with respect to each other. The device 1 is integrally formed using conventional reel-to-reel technology as detailed in the introductory portion. The stacked structure of the device 1 generally includes a first portion 2 and a second portion 3 in fluid communication, i.e., in contact with the first portion 2.

Specifically, the first portion 2 includes a take-up layer 4 for taking-up the blood sample made of flexible foamed material such as, but not limited to, open-cellular foamed plastic, e.g., made of melamine resin. The take-up layer 4 preferably is of rectangular, in particular quadratic shape having an edge length of about 10 mm and a thickness of about 1.5 mm.

The first portion 2 further includes a depth filtration layer 5 adjacent the take-up layer 4 and in series with a plasma separation membrane 6. The depth filtration layer 5 is adapted to slow the flow of blood cells relative to that of the plasma in order to avoid clogging of the plasma separation membrane 6. It further enables a lateral diffusion of the blood components to thereby broaden the blood-contacted area of the plasma-separation membrane 6. The depth filtration layer 5 preferably is made of glass fibre material and is of rectangular, in particular quadratic shape having an edge length of about 14 mm and a thickness of about 1 mm.

The plasma separation membrane 6 is adapted to permit plasma flow and block the passage of the cellular components of the blood, i.e. red and white blood cells and platelets. It preferably is made of porous filter material having pores adapted to capture the cellular components of the blood and preferably is of rectangular, in particular quadratic shape having an edge length of about 14 mm and a thickness of much smaller than 1 mm. In the introductory portion, the depth filtration layer 5 and the plasma separation membrane 6 together are denoted as "separating member".

The second portion 3 includes an absorptive (wicking) layer 7 adjacent and in contact with the plasma separation membrane 6 for absorbing plasma by capillary pressure thus acting as suction pump for sucking blood through the separating member. The absorptive layer 7 preferably is made of porous filter paper and is of rectangular, in particular quadratic shape having an edge length of about 12 mm and a thickness of about 1 mm.

The second portion 3 further includes a backing layer 8 adjacent the absorptive layer 7 for backing the absorptive layer 7 (film) which, e.g., can be made of stiff polyethylenterephtalate (PET) material and preferably is of rectangular, in particular quadratic shape having an edge length of about 12 mm and a thickness of about 500 µm. As illustrated in Fig. 1, the backing layer 8 can, e.g., be provided with a recess 11 accommodating the absorptive layer 7. In case of a lateral offset between the first and second portions 2, 3, the lateral diffusion of the blood plasma caused by the depth-filtration layer 5 advantageously enables that an increased amount of plasma can be absorbed by the absorptive layer 7.

The first portion 2 is removably fixed to the second portion 3 by a middle adhesive element 12 which, e.g., can be an adhesive layer consisting of adhesive material or may include a supporting layer provided on one or both sides with an adhesive layer made of adhesive material in such a manner that the first portion 2 can be drawn away or peeled off from the second portion 3 without destruction of the second portion 3, in particular, without destroying the absorptive layer 7. Accordingly, the first portion 2 can be removed from the second portion 3 by breaking (disconnecting) the adhesive element 12, the adhesive element 12 thus serving as predetermined breaking zone.

In the device 1, the stacked structure further includes a cover layer 9 arranged on top of the stacked structure above the take-up layer 4 and a bottom layer 10 at the bottom-side of the backing layer 8, both of which can be made of non-elastic plastic materials. Specifically, the cover layer 9 is fixed to the take-up layer 4 by an upper adhesive element 13 while the bottom layer 10 is fixed to both the backing layer 8 and the absorptive layer 7 by a lower adhesive element 14. Preferably the upper and lower adhesive elements 13, 14 are adhesive films made of adhesive material. More specifically, the bottom layer 10 and the backing layer 8 together are adapted to keep the absorptive layer 7 in a predetermined position with respect to the stacked arrangement.

In the device 1, the take-up layer 4 is made of an elastically compressible material and arranged in-between the cover layer 9 and the depth filtration layer 5 in a (pre-) compressed condition so that the non-elastic cover layer 9 and the non-elastic bottom layer 10 fixed to the cover layer 9 together take up the elastic (de-)compression force of the take-up layer 4.

As a result, the depth filtration layer 5, the plasma separation layer 6 and the absorptive layer 7 are forced against the bottom layer 10 which assures a close fit between the various stacked layers of the device 1. Stated more particularly, the depth filtration layer 5 is provided with a depth filtration layer surface 16 for receiving blood from the take-up layer 4 which is in close contact with full fit for fluid communication between the depth filtration layer 5 and the take-up layer 4. The plasma separation membrane 6 is provided with a plasma separation membrane surface 17 for receiving blood from the depth filtration layer 16 which is in close contact with full fit for fluid communication between the plasma separation membrane 6 and the depth filtration layer 5. The absorptive layer 7 is provided with an absorptive layer surface 18 for receiving plasma from the plasma separation membrane 6 which is in close contact with full fit for fluid communication between the absorptive layer 7 and the plasma separation membrane 6. Otherwise, the take-up layer 4 is provided with a take-up layer surface 15 for applying whole blood through an opening 19 of the cover layer 9. Accordingly, an intense capillary force can act on the blood sample applied on the take-up layer surface 15 to draw it through the depth filtration layer 5 and the plasma separation membrane 6 for plasma separation and absorption in the absorptive layer 7. Since the absorptive layer 7 has a plasma capacity which is less than the amount of plasma in the blood volume contained in the separating member comprised of the depth filtration layer 5 and the plasma separation membrane 6, the absorptive layer 7 becomes saturated with a predetermined amount of plasma when drawing at least a portion or all of the plasma contained in the separating member into the absorptive layer 7. The separating member preferably contains a plasma-soluble dye so as to signalize a plasma-saturated condition of the absorptive layer 7.

Using the device 1 of Fig. 1, a process for separating plasma from a blood sample starts with applying the whole blood on the take-up layer surface 15, e.g., in pricking a patient's finger and tipping onto the take-up layer surface 15 until a predetermined volume of the blood of, say, 150 µl is soaked into the take-up layer 4.

After waiting for a predetermined time span of, e.g., a few minutes, it is checked whether the absorptive layer 7 is completely filled (saturated) with plasma, e.g., using a plasma-soluble dye as-above detailed. Upon reaching plasma saturation, the second portion 3 is peeled off from the first portion 2 in a wet condition of the absorptive layer 7 so as to separate the absorptive layer 7 from the separating member comprised of the depth filtration layer 5 and the plasma separation membrane 6. By separating the absorptive layer 7 from the separating member in a wet condition, several favourable effects can be obtained: a first effect of hindering diffusion of plasma from the absorptive layer 7 towards the separating member; a second effect of hindering decomposition products due to hemolysis of residual blood components in the separating member to get into the absorptive layer 7 which can strongly influence subsequent plasma analysis; and a third effect of hindering undesired sticking of the plasma separation membrane 6 and the adsorptive layer 7 which can result in parts of the plasma separation membrane 6 adhering on the absorptive layer 7.

Then, the absorptive layer 7 is dried for a predetermined time interval of, e.g., a few hours. A humidity detector (not illustrated) which is operatively coupled to the absorptive layer 7 can be used to detect humidity of the absorptive layer 7 for outputting at least two optical and/or acoustical signals which are different with respect to each other in response to a detection result, wherein a first signal signifies a dried condition and a second signal a wet condition of the absorptive layer 7.

The second portion 3 including the dried absorptive layer 7 backed by the backing layer 8 can be packaged, preferably as card or vial on desiccant for shipping at room temperatures to a specific analysis site for plasma analysis. Specifically, the backing layer 8 preferably is provided with a machine-readable label such as a barcode which advantageously allows for an easy, quick and cost-effective identification at the analysis site. The machine-readable label can, e.g., be disposed on a back-side of the backing layer 8, i.e., on a lower backing layer surface of the backing layer 8. At the analysis site, the dried plasma is dissolved and diluted in an elution medium to perform specific tests which can be related to substances which are characteristic of specific infections such as HIV particles, e.g., involving the use of the polymerase chain reaction (PCR) or any other technique of the nucleic acid amplification type. Specifically, the backing layer 8 can be made of a material which can be dissolved in the elution medium eliminating a risk of clotting in pipetting operations.

With particular reference to Fig. 2 which is a schematic sectional view another exemplary embodiment of the device 1 according to the invention is explained. In order to avoid unnecessary repetitions, only differences with respect to the embodiment of Fig. 1 are explained and, otherwise, reference is made thereto.

Accordingly, in the device 1, the second portion 3 is a structural entity comprised of a solid inert backing layer 8 wherein the absorptive layer 7 is arranged on an upper backing layer surface 20 of the backing layer 8. The absorptive layer 7 is removably fixed to the upper backing layer surface 20 of the backing layer 8 by a first adhesive spot 21, e.g., made of a brittle glue arranged in a middle portion of the absorptive layer 7 so that the absorptive layer is partly fixed to the backing layer 8 having non-fixed end region. In the introductory portion, the first adhesive spot 21 is denoted as second adhesive element. A second handle 24 is fixed to the absorptive layer 7 at an (non-fixed) edge region thereof so that the absorptive layer 7 can be readily removed from the backing layer 8 by manually gripping the second handle 24. While not shown in Fig. 2, only the end-region of the absorptive layer 7 connected to the second handle 24 can be non-fixed to the backing layer 8 while the other end-region is fixed to the backing layer 8 by means of the first adhesive spot 21.

Similarly, the first portion 2 is a structural entity comprising the depth filtration layer 5 and the plasma separation membrane 6. While not shown in FIG. 2, the first portion 2 may also include the take-up layer 4. The first portion 2 is removably fixed to the backing layer surface 20 by means of a second adhesive spot 22, e.g., made of a brittle glue at an edge region of the first portion 2. In the introductory portion, the second adhesive spot 21 is denoted as first adhesive element.

Accordingly, both the first portion 2 and the second portion 3 are removably fixed to the upper backing layer surface 20 of the backing layer 8. Specifically, the first portion 2 partly overlaps the second portion 3 in a region where the second portion is fixed to the backing layer 8. Accordingly, a first overlapping region 27 is formed which has a stacked configuration in which the first portion 2 is located above the second portion 3. Thus, the first portion 2 is arranged to enable fluid communication with the second portion 3 so that plasma can be transported from the first portion 2 to the absorptive layer 7. Accordingly, the first portion 2 or at least a part thereof is in contact with the second portion 3. Otherwise, the first portion 2 or at least a part thereof can be brought in contact with the second portion 3 in such a way that plasma transfer is possible, e.g., by the pressing the first portion 2 in the direction of the backing layer 8. Furthermore, a first handle 23, opposite to the second handle 24, is fixed to the first portion 2 by means of the second adhesive spot 22 so that it can be readily removed from the backing layer 8 by manually gripping the first handle 23.

As illustrated in Fig. 2, a sample of whole blood 25 can be applied to the depth filtration layer surface 16 of the first overlapping region 27 which then is drawn through the first portion 2 to separate plasma 26 into the absorptive layer 7 by capillary pressure. Then, the first portion 2 is removed from the second portion 3 in a wet condition of the absorptive layer 7 by manual interaction at the first handle 23, followed by drying the absorptive layer 7 and shipping the second portion 3 to an analysis site in a dried condition. The absorptive layer 7 can be removed from the backing layer 8 by manual interaction of the second handle 24, if desired, e.g., at the analysis site facilitating plasma analysis. Stated more particularly, the absorptive layer 7 can, e.g., be removed from the backing layer 8 by bending the second portion 3 so that it can be helpful to have sufficient plasma-free space on both ends of the second portion 3 which allows for gripping and bending. Bending can also be reached by jamming the second portion 3 into a tube having an S-shaped inlet to thereby lose the absorptive layer 7 that can be left in the tube while removing the backing layer 8. Otherwise, the second portion 3 can have a predetermined breaking point which can be activated by inserting it in a suitable tube or, alternatively, can have a predetermined part for cutting or punching off. Obviously, the device 1 of Fig. 2 can be manufactured in a very easy and cost-effective manner in large numbers.

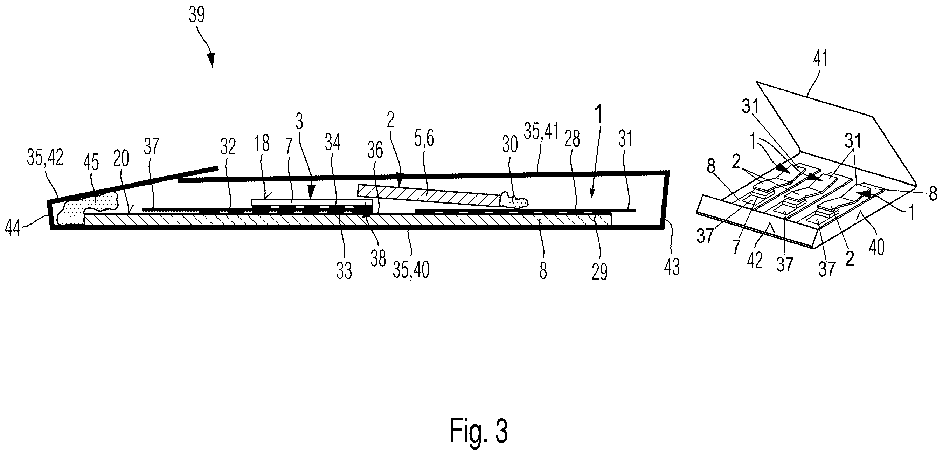

With particular reference to Fig. 3 which depicts a schematic sectional view and a small perspective view a yet another exemplary embodiment of the device 1 according to the invention is explained. In order to avoid unnecessary repetitions, only differences with respect to the embodiment of Fig. 1 are explained and, otherwise, reference is made thereto.

Accordingly, the device 1 includes the first portion 2 made up of the depth filtration layer 5 and the plasma separation membrane 6 and the second portion 3 made up of the absorptive layer 7, wherein both the first and second portions 2,3 are fixed to the upper backing layer surface 20 of the backing layer 8. While not shown in FIG. 3, the first portion 2 may also include the take-up layer 4.

Stated more particularly, the first portion 2 is fixed to the upper side of a first carrier or supporting layer 28 by means of a third adhesive spot 30 made of adhesive material such as brittle glue at an edge region of the first portion 2. The third adhesive spot 30 is located in a middle portion of the first supporting layer 28. On its lower side, the first supporting layer 28 is removably fixed to the upper backing layer surface 20 of the backing layer 8 by means of a peelable lower first adhesive coating 29 so that the first supporting layer 28 together with the first portion 2 can be peeled off from the backing layer 8 without destroying the absorptive layer 7, the first adhesive coating 29 thereby serving as predetermined breaking zone.

The first supporting layer 28 which preferably is of rectangular shape extends well beyond the backing layer 8 to thereby form a first gripping portion 31 for manually gripping the first supporting layer 28. The first gripping portion 31 is free of the lower first adhesive coating 29. Accordingly, the lower first adhesive coating 29 covers only a part of the first supporting layer 28 so that the first supporting layer 28 can be readily peeled off from the backing layer 8.

The second portion 3, i.e., the absorptive layer 7, is removably fixed to a second supporting layer 32 by means of a peelable upper second adhesive coating 34 made of adhesive material. The second supporting layer 32 is located adjacent the first supporting layer 28 leaving a small gap 36 between the first and second supporting layers 28, 32. On its lower side, the second supporting layer 32 is removably fixed to the backing layer 8 by means of a lower second adhesive coating 33 made of adhesive material so that the second supporting layer 32 together with the absorptive layer 7 can be peeled off from the backing layer 8 without destroying the absorptive layer 7, the lower second adhesive coating 33 thereby serving as predetermined breaking zone(s). Otherwise, the absorptive layer 7 can be removed from the upper second adhesive coating 34 without destroying the absorptive layer 7.

The second supporting layer 32 which preferably is of rectangular shape extends well beyond the second portion 3 to thereby form a second gripping portion 37 for manually gripping the second supporting layer 32. While the lower second adhesive coating 33 extends beyond the second portion 3, the second gripping portion 37 is free of the second adhesive coatings 33, 34 so that the second supporting layer 32 can be readily peeled off from the backing layer 8. Accordingly, the second adhesive coatings 33, 34 cover only a part of the second supporting layer 32. While the second gripping portion 37 is in a flat position on the backing layer 8, it can be readily lift off for gripping, e.g., by means of a finger's nail.

The first portion 2 extends towards the second portion 3 bridging the gap 36 and partly overlaps the second portion 3 in a region where the second portion 3 is fixed to the backing layer 8. Accordingly, a second overlapping region 38 is formed which has a stacked configuration in which the first portion 2 is located above the second portion 3 to be or to be brought in fluid communication (i.e. in contact) therewith so that plasma can be transported from the first portion 2 to the absorptive layer 7. Accordingly, the first portion 2 is positioned so that at least a part thereof is or can be brought into contact with the second portion 3 in such a way that plasma transfer is possible by the pressing the first portion 2 in the direction of the backing layer 8.

In an arrangement, generally referred to at reference numeral 39, an envelope 35 preferably made of paper or papercoard contains one or more devices 1. While the arrangement 39 is shown to contain three devices 1 in serial arrangement with respect to each other, those of skill in the art will appreciate that the arrangement 39 may also contain a larger or smaller number of devices 1. The envelope 35 includes a bottom portion 40 and a larger first top portion 41 and a smaller second top portion 42 connected to the bottom portion 40 by first and second connecting portions 43, 44, respectively, arranged in opposite relationship with respect to each other. The backing layer 8 is fixed to the envelope 35 by means of a fourth adhesive spot 45 made of adhesive material such as brittle glue at an edge region adjacent the second connecting portion 44 of the envelope 35.

The first and second top portions 41, 42 can be opened or closed according to the specific demands of the user, e.g., by bending the connecting portions 43, 44, wherein in closed positions the top portions 41, 42 overlap each other so that the envelope 35 fully covers the devices 1. Stated more particularly, the larger first top portion 41 covers a major part of the devices 1 including the second overlapping region 38. Otherwise, the smaller second top portion 42 covers a minor part of the devices 1 including the second gripping portion 37.

Accordingly, in opened position of the larger first top portion 41, while keeping the smaller second top portion 42 in closed position to thereby hide the second gripping portion 37, a sample of whole blood can be applied to the depth filtration layer surface 16 of the second overlapping region 38 of one or more devices 1. The blood is then drawn through the first portion 2 into the absorptive layer 7 by capillary pressure. Then, the first portion 2 is removed from the second portion 3 in a wet condition of the absorptive layer 7 by manual interaction at the first gripping portion 31, followed by drying the absorptive layer 7 and shipping the arrangement 39 having the larger first top portion 41 in closed position to an analysis site in a dried condition. At the analysis site, the absorptive layer 7 can be readily removed from the backing layer 8 by manual interaction at the second gripping portion 37 facilitating plasma analysis. Accordingly, the second gripping portion 37 is advantageously hide by the smaller first top portion 41 in closed position when applying the blood sample so that inadvertent manual gripping of the second gripping portion 37, e.g., by non-professional (non-medical) users can be avoided. Otherwise, the arrangement 39 can be safely shipped to the analysis site having the first and second top portions 41, 42 in closed positions to avoid contamination. At the analysis site, the smaller second top portion 42 is opened to unhide the second gripping portion by professional (medical) users for manual gripping so as to remove the absorptive layer 7 from the backing layer 8.

Obviously many modifications and variations of the present invention are possible in light of the above description. It is therefore to be understood, that within the scope of appended claims, the invention may be practiced otherwise than as specifically devised.

### Reference list

- 1: Device
- 2: First portion
- 3: Second portion
- 4: Take-up layer
- 5: Depth filtration layer
- 6: Plasma separation membrane
- 7: Absorptive layer
- 8: Backing layer
- 9: Top layer
- 10: Bottom layer
- 11: Recess
- 12: Middle adhesive element
- 13: Upper adhesive element
- 14: Lower adhesive element
- 15: Take-up layer surface
- 16: Depth filtration layer surface
- 17: Plasma separation membrane surface
- 18: Absorptive layer surface
- 19: Opening
- 20: Backing layer surface
- 21: First adhesive spot
- 22: Second adhesive spot
- 23: First handle
- 24: Second handle
- 25: Blood
- 26: Plasma
- 27: First overlapping region
- 28: First supporting layer
- 29: Lower first adhesive coating
- 30: Third adhesive spot
- 31: First gripping portion
- 32: Second supporting layer
- 33: Lower second adhesive coating
- 34: Upper second adhesive coating
- 35: Envelope
- 36: Gap
- 37: Second gripping portion
- 38: Second overlapping region
- 39: Arrangement
- 40: Bottom portion
- 41: First top portion
- 42: Second top portion
- 43: First connecting portion
- 44: Second connecting portion
- 45: Fourth adhesive spot

## Claims

1. A device (1) for separating plasma (26) from a blood sample (25), said blood sample including a partial volume of plasma and a partial volume of cellular components, said device comprising:
a first portion (2) including a separating member (5, 6) having a first surface (16) for applying or receiving said blood sample (25), said separating member (5, 6) being adapted to permit the passage of plasma but to inhibit the passage of cellular components,
a second portion (3) having an absorptive member (7) for absorbing plasma and a backing member (8) arranged in a manner to support said absorptive member (7), said absorptive member (7) having a second surface (18) in contact with said separating member (5, 6) for receiving plasma and being adapted to generate a capillary pressure so as to draw plasma from said separating member (5, 6) to said absorptive member (7),
wherein said first portion (2) is fixed to said backing member (8) by means of at least one first adhesive element (22; 29) in such a manner that said first portion (2) can be removed from said backing member (8) by selectively breaking the first adhesive element (22; 29) without destroying said absorptive member (7), and
wherein said absorptive member (7) is fixed to said backing member (8) by means of at least one second adhesive element (21; 33) in such a manner that said absorptive member (7) can be non-destructively removed from said backing member (8) by selectively breaking the second adhesive element (22; 29),
the device further comprising:
at least one first gripping means (24; 37) fixed to the absorptive member (7) adapted for removing said absorptive member (7) from said backing member (8).

2. The device (1) according to claim 1, wherein said first portion (2) is fixed to a first supporting layer (28) provided with an adhesive layer (29) for fixing to said backing member (8), said adhesive layer (29) being adapted for peeling off said first supporting layer (28) from said backing member (8).

3. The device (1) according to claims 1 or 2, wherein said absorptive member (7) is fixed to a second supporting layer (32) provided with an adhesive layer (33) for fixing to said backing member (8), said adhesive layer (29) being adapted for peeling off said second supporting layer (32) from said backing member (8).

4. The device (1) according to claim 1, wherein each of said first and second adhesive elements is configured as an adhesive zone (22, 21) for fixing said first portion (2) and said absorptive member (7), respectively, to said backing member (8).

5. The device (1) according to any one of the preceding claims 1 to 4, including at least one second gripping means (23; 31), adapted for removing said first portion (2) from said backing member (8).

6. The device (1) according to any one of the preceding claims 1 to 5, wherein said first portion (2) includes a take-up member (4) having a third surface (15) for applying said blood sample (25), said first surface (16) of said separating member (5, 6) being in contact with said take-up member (4) for receiving said blood sample by capillary pressure generated by said absorptive member (7).

7. The device (1) according to any one of the preceding claims 1 to 6, including a cover member (9) arranged on top of the first portion (2) covering at least a portion thereof, said cover member (9) being provided with an opening (19) for applying said blood sample and being fixed to said second portion (3) and/or a bottom member (10) arranged at the bottom-side of the second portion (3).

8. The device (1) according to claim 7, wherein said take-up member (4) is made of an elastically compressible material and is arranged in-between said cover member (9) and said separating member (5, 6) in a compressed condition.

9. The device (1) according to any one of the preceding claims 1 to 7, wherein said second portion (3), in particular said backing layer (8), is provided with a machine-readable label such as a barcode.

10. The device (1) according to any one of the preceding claims 1 to 9, wherein said absorptive member (7) is operatively coupled to a humidity detector adapted to detect humidity of said absorptive member (7).

11. The device (1) according to any one of the preceding claims 1 to 10, wherein said absorptive member (7) is made of a material adapted to be dissolved in an elution medium for eluting dried plasma.

12. The device (1) according to any one of the preceding claims 1 to 11, wherein said separating member (5, 6) contains a plasma-soluble dye.

13. A process for separating plasma from a blood sample comprising the following steps of:
- providing a device according to claims 1-12;
- applying said blood sample to the separating member (5, 6) adapted to permit the passage of plasma but to inhibit the passage of blood cells;
- drawing said blood sample through said separating member (5, 6) to the absorptive member (7) for absorbing plasma by means of capillary pressure generated by said absorptive member (7);
- non-destructively removing the first portion (2) from the backing member (8) by selectively breaking the first adhesive element (22; 29) fixing the first portion (2) to the backing member (8) without destroying said absorptive member (7);
- non-destructively removing said absorptive member (7) from said backing member (8) by selectively breaking a second adhesive element (22; 29) fixing the absorptive member (7) to the backing member (8) by using at least one first gripping means (24; 37) fixed to the absorptive member (7) adapted for removing said absorptive member (7) from said backing member (8).

## Patentansprüche

1. Vorrichtung (1) zum Abtrennen von Plasma (26) aus einer Blutprobe (25), wobei die Blutprobe ein Teilvolumen von Plasma und ein Teilvolumen zellulärer Bestandteile aufweist, wobei die Vorrichtung das Folgende umfasst:
einen ersten Abschnitt (2) mit einem Trennelement (5, 6) mit einer ersten Oberfläche (16) zum Auftragen oder Empfangen der Blutprobe (25), wobei das Trennelement (5, 6) dazu angepasst ist, das Durchleiten von Plasma zu erlauben, aber das Durchleiten von zellulären Bestandteilen zu unterbinden,
einen zweiten Abschnitt (3) mit einem saugfähigen Element (7) zum Aufsaugen von Plasma und einem Trägerelement (8), das derart angeordnet ist, dass es das saugfähige Element (7) unterstützt, wobei das saugfähige Element (7) eine zweite Oberfläche (18) in Kontakt mit dem Trennelement (5, 6) zum Aufnehmen von Plasma aufweist und dazu angepasst ist, einen Kapillardruck zu erzeugen, um Plasma vom Trennelement (5, 6) zum saugfähigen Element (7) zu ziehen,
wobei der erste Abschnitt (2) mit mindestens einem ersten Klebeelement (22; 29) derart am Trägerelement (8) befestigt ist, dass der erste Abschnitt (2) durch selektives Zerbrechen des ersten Klebeelements (22; 29) ohne Zerstörung des saugfähigen Elements (7) vom Trennelement (8) entfernt werden kann, und
wobei das saugfähige Element (7) mit mindestens einem zweiten Klebeelement (21; 33) derart am Trägerelement (8) befestigt ist, dass das saugfähige Element (7) durch selektives Zerbrechen des zweiten Klebeelements (22; 29) ohne Zerstörung vom Trennelement (8) entfernt werden kann,
wobei die Vorrichtung ferner Folgendes umfasst:
mindestens ein erstes Greifmittel (24; 37), das am saugfähigen Element (7) befestigt ist und zur Entfernung des saugfähigen Elements (7) vom Trägerelement (8) angepasst ist.

2. Vorrichtung (1) nach Anspruch 1, wobei der erste Abschnitt (2) an einer ersten Stützschicht (28), die eine Klebeschicht (29) zur Befestigung am Trägerelement (8) aufweist, befestigt ist, wobei die Klebeschicht (29) zum Abziehen der ersten Stützschicht (28) vom Trägerelement (8) angepasst ist.

3. Vorrichtung (1) nach den Ansprüchen 1 oder 2, wobei das saugfähige Element (7) an einer zweiten Stützschicht (32), die eine Klebeschicht (33) zur Befestigung am Trägerelement (8) aufweist, befestigt ist, wobei die Klebeschicht (29) zum Abziehen der zweiten Stützschicht (32) vom Trägerelement (8) angepasst ist.

4. Vorrichtung (1) nach Anspruch 1, wobei die ersten und zweiten Klebeelemente jeweils als Klebezone (22, 21) zur Befestigung des ersten Abschnitts (2) bzw. des saugfähigen Elements (7) am Trägerelement (8) ausgelegt sind.

5. Vorrichtung (1) nach einem der vorhergehenden Ansprüche 1 bis 4, umfassend mindestens ein zweites Greifmittel (23; 31), das zur Entfernung des ersten Abschnitts (2) vom Trägerelement (8) angepasst ist.

6. Vorrichtung (1) nach einem der vorhergehenden Ansprüche 1 bis 5, wobei der erste Abschnitt (2) ein Aufnahmeelement (4) mit einer dritten Oberfläche (15) zum Auftragen der Blutprobe (25) aufweist, wobei die erste Oberfläche (16) des Trennelements (5, 6) mit dem Aufnahmeelement (4) in Kontakt ist, um die Blutprobe durch einen vom saugfähigen Element (7) erzeugten Kapillardruck zu empfangen.

7. Vorrichtung (1) nach einem der vorhergehenden Ansprüche 1 bis 6, umfassend ein Abdeckelement (9), das auf der Oberseite des ersten Abschnitts (2) angeordnet ist und mindestens einen Teil davon abdeckt, wobei das Abdeckelement (9) eine Öffnung (19) zum Auftragen der Blutprobe aufweist und am zweiten Abschnitt (3) und/oder einem Bodenelement (10), das an der Bodenseite des zweiten Abschnitts (3) angeordnet ist, befestigt ist.

8. Vorrichtung (1) nach Anspruch 7, wobei das Aufnahmeelement (4) aus einem elastisch komprimierbaren Material gefertigt ist und zwischen dem Abdeckelement (9) und dem Trennelement (5, 6) in einem komprimierten Zustand angeordnet ist.

9. Vorrichtung (1) nach einem der vorhergehenden Ansprüche 1 bis 7, wobei der zweite Abschnitt (3), insbesondere die Trägerschicht (8), ein maschinenlesbares Etikett, wie etwa einen Strichcode, aufweist.

10. Vorrichtung (1) nach einem der vorhergehenden Ansprüche 1 bis 9, wobei das saugfähige Element (7) funktionsfähig mit einem Feuchtigkeitsdetektor verbunden ist, der zum Nachweisen von Feuchtigkeit des saugfähigen Elements (7) angepasst ist.

11. Vorrichtung (1) nach einem der vorhergehenden Ansprüche 1 bis 10, wobei das saugfähige Element (7) aus einem Material gefertigt ist, das dazu angepasst ist, in einem Elutionsmedium zum Eluieren von getrocknetem Plasma gelöst zu werden.

12. Vorrichtung (1) nach einem der vorhergehenden Ansprüche 1 bis 11, wobei das Trennelement (5, 6) einen plasmalöslichen Farbstoff enthält.

13. Verfahren zum Abtrennen von Plasma aus einer Blutprobe, umfassend die folgenden Schritte:
- Bereitstellen einer Vorrichtung nach einem der Ansprüche 1-12;
- Auftragen der Blutprobe auf das Trennelement (5, 6), das zum Durchleiten von Plasma, aber zum Unterbinden des Durchleitens von Blutzellen angepasst ist;
- Ziehen der Blutprobe durch das Trennelement (5, 6) zum saugfähigen Element (7) zum Absorbieren von Plasma mittels eines vom saugfähigen Element (7) erzeugten Kapillardrucks;
- zerstörungsfreies Entfernen des ersten Abschnitts (2) vom Trägerelement (8) durch selektives Zerbrechen des ersten Klebeelements (22; 29), das den ersten Abschnitt (2) am Trägerelement (8) befestigt, ohne das saugfähige Element (7) zu zerstören;
- zerstörungsfreies Entfernen des saugfähigen Elements (7) vom Trägerelement (8) durch selektives Zerbrechen eines zweiten Klebeelements (22; 29), das das saugfähige Element (7) am Trägerelement (8) befestigt, durch Verwendung mindestens eines Greifmittels (24; 37), das am saugfähigen Element (7) befestigt ist und zum Entfernen des saugfähigen Elements (7) vom Trägerelement (8) angepasst ist.

## Revendications

1. Dispositif (1) de séparation de plasma (26) d'un échantillon de sang (25), ledit échantillon de sang comprenant un volume partiel de plasma et un volume partiel de composants cellulaires, ledit dispositif comprenant :
une première partie (2) comprenant un élément de séparation (5, 6) possédant une première surface (16) pour appliquer ou recevoir ledit échantillon de sang (25), ledit élément de séparation (5, 6) étant conçu pour permettre le passage de plasma, mais pour empêcher le passage de composants cellulaires,
une seconde partie (3) possédant un élément d'absorption (7) pour absorber le plasma et un élément de renfort (8) agencé de manière à supporter ledit élément d'absorption (7), ledit élément d'absorption (7) possédant une deuxième surface (18) en contact avec ledit élément de séparation (5, 6) pour recevoir du plasma et étant conçu pour générer une pression capillaire de façon à aspirer le plasma dudit élément de séparation (5, 6) vers ledit élément d'absorption (7),
dans lequel ladite première partie (2) est fixée audit élément de renfort (8) au moyen d'au moins un premier élément adhésif (22 ; 29) de sorte que ladite première partie (2) puisse être retirée dudit élément de renfort (8) en rompant sélectivement le premier élément adhésif (22 ; 29) sans détruire ledit élément d'absorption (7), et
dans lequel ledit élément d'absorption (7) est fixé audit élément de renfort (8) au moyen d'au moins un second élément adhésif (21 ; 33) de sorte que ledit élément d'absorption (7) puisse être retiré de manière non destructrice dudit élément de renfort (8) en rompant sélectivement le second élément adhésif (22 ; 29),
le dispositif comprenant en outre :
au moins un premier moyen de préhension (24 ; 37) fixé à l'élément d'absorption (7) conçu pour retirer ledit élément d'absorption (7) dudit élément de renfort (8).

2. Dispositif (1) selon la revendication 1, dans lequel ladite première partie (2) est fixée à une première couche de support (28) dotée d'une couche adhésive (29) pour une fixation audit élément de renfort (8), ladite couche adhésive (29) étant conçue pour décoller ladite première couche de support (28) dudit élément de renfort (8).

3. Dispositif (1) selon la revendication 1 ou 2, dans lequel ledit élément d'absorption (7) est fixé à une seconde couche de support (32) dotée d'une couche adhésive (33) pour une fixation audit élément de renfort (8), ladite couche adhésive (29) étant conçue pour décoller ladite seconde couche de support (32) dudit élément de renfort (8).

4. Dispositif (1) selon la revendication 1, dans lequel chacun desdits premier et second éléments adhésifs est conçu en tant que zone adhésive (22, 21) pour fixer ladite première partie (2) et ledit élément d'absorption (7), respectivement, audit élément de renfort (8).

5. Dispositif (1) selon l'une quelconque des revendications précédentes 1 à 4, comprenant au moins un second moyen de préhension (23 ; 31), conçu pour retirer ladite première partie (2) dudit élément de renfort (8).

6. Dispositif (1) selon l'une quelconque des revendications précédentes 1 à 5, dans lequel ladite première partie (2) comprend un élément récepteur (4) possédant une troisième surface (15) pour appliquer ledit échantillon de sang (25), ladite première surface (16) dudit élément de séparation (5, 6) étant en contact avec ledit élément récepteur (4) pour recevoir ledit échantillon de sang par une pression capillaire générée par ledit élément d'absorption (7).

7. Dispositif (1) selon l'une quelconque des revendications précédentes 1 à 6, comprenant un élément de recouvrement (9) agencé en haut de la première partie (2) recouvrant au moins une partie de celle-ci, ledit élément de recouvrement (9) étant doté d'une ouverture (19) pour appliquer ledit échantillon de sang et étant fixé à ladite seconde partie (3) et/ou à un élément inférieur (10) agencé au niveau du côté inférieur de la seconde partie (3).

8. Dispositif (1) selon la revendication 7, dans lequel ledit élément récepteur (4) est en matériau compressible élastiquement et est agencé entre ledit élément de recouvrement (9) et ledit élément de séparation (5, 6) dans un état comprimé.

9. Dispositif (1) selon l'une quelconque des revendications précédentes 1 à 7, dans lequel ladite seconde partie (3), en particulier ladite partie de renfort (8), est pourvue d'une étiquette lisible par machine telle qu'un code-barres.

10. Dispositif (1) selon l'une quelconque des revendications précédentes 1 à 9, dans lequel ledit élément d'absorption (7) est accouplé fonctionnellement à un détecteur d'humidité conçu pour détecter l'humidité dudit élément d'absorption (7).

11. Dispositif (1) selon l'une quelconque des revendications précédentes 1 à 10, dans lequel ledit élément d'absorption (7) est en matériau conçu pour être dissous dans un milieu d'élution pour éluer un plasma séché.

12. Dispositif (1) selon l'une quelconque des revendications précédentes 1 à 11, dans lequel ledit élément de séparation (5, 6) contient un colorant soluble dans le plasma.

13. Procédé de séparation du plasma d'un échantillon de sang comprenant les étapes suivantes consistant à :
- fournir un dispositif selon les revendications 1 à 12 ;
- appliquer ledit échantillon de sang à l'élément de séparation (5, 6) conçu pour permettre le passage du plasma, mais pour empêcher le passage des cellules sanguines ;
- aspirer ledit échantillon de sang à travers ledit élément de séparation (5, 6) vers l'élément d'absorption (7) pour absorber le plasma au moyen d'une pression capillaire générée par ledit élément d'absorption (7) ;
- retirer de manière non destructrice la première partie (2) de l'élément de renfort (8) en rompant sélectivement le premier élément adhésif (22 ; 29) fixant la première partie (2) à l'élément de renfort (8) sans détruire ledit élément d'absorption (7) ;
- retirer de manière non destructrice ledit élément d'absorption (7) dudit élément de renfort (8) en rompant sélectivement un second élément adhésif (22 ; 29) fixant l'élément d'absorption (7) à l'élément de renfort (8) en utilisant au moins un premier moyen de préhension (24 ; 37) fixé à l'élément d'absorption (7) conçu pour retirer ledit élément d'absorption (7) dudit élément de renfort (8).
